# EUROPEAN PATENT APPLICATION

(11) **EP 3 881 837 A1**
(43) Date of publication of application: **22.09.2021**
(21) Application number: 21163117.1
(22) Date of filing: 17.03.2021
(51) Int. Cl.: A61K 31/015, A61P 17/02

(54) **PHARMACEUTICAL COMPOSITION FOR THE BACTERIAL BIOFILM REMOVAL**

(30) Priority: 18.03.2020 IT 202000005731
(71) Applicant: Dapa S.r.l., 37135 Verona VR (IT)
(72) Inventor: Caobelli, Daniele, 37135 Verona VR (IT); Menegazzi, Giampaolo, 37135 Verona VR (IT)
(74) Representative: Perani & Partners S.p.A.

(57) **Abstract**

Pharmaceutical composition for use as a medicament comprising one or more compounds of formula (I), wherein at least one of the substituents R1, R2 and R3 is selected from the group consisting of: straight or branched C1-C4 alkyl groups, C1-C3 chain aliphatic ethers, aromatic ethers and halogens, and the remaining substituent(s) among R1, R2 and R3 is hydrogen. A further object of the invention is a kit comprising the pharmaceutical composition and devices suitable for the application and dosage thereof.

## Description

### FIELD OF THE INVENTION

The present invention relates to a pharmaceutical composition for use as a medicament, in particular for disinfecting skin and mucosal ulcers.

### STATE OF THE ART

A large amount of human infections develops by forming a particular biological system called "biofilm". A biofilm is composed of groups of different microorganisms contained within a self-produced matrix of extracellular polymeric substance consisting of extracellular DNA, proteins and polysaccharides. Within the biofilm, some bacteria can modulate the metabolism thereof by becoming extremely active and resistant to the action of many antibacterial agents.

The microorganisms (the microbiota) which constitute the human microbiome are not only single-celled organisms living side by side, but form highly regulated communities, structurally and functionally linked to tissue surfaces in the form of biofilms, with inter-species collaborations and antagonisms which contribute to the stability of the microbiome.

The bacterial interactions of the skin and ulcers are important in modifying pathophysiological mechanisms contributing to delayed healing. In some diseases, for example diabetes, ulcers tend to have a slow and difficult healing which, if not resolved and sustained, can lead to poor prognoses for the patient (in the case of the diabetic foot, amputation).

When the biofilm develops, the bacteria demonstrate greater resistance to the various immune mechanisms put in place by the host, as well as to antibiotics, and the biofilm-producing bacteria have greater virulence. The sequence of events which occur during the formation of the biofilm is complex: it begins with a random bacterial settlement (first colonizers) which determines a competition between the various species with the prevalence of those able to survive in the niche as biofilm producers. The microbiota of diabetic ulcers is organized to act in synergy and determine a chronic infection. Vigorous mechanical debridement (removal of torn, devitalized or contaminated tissue) is required to resolve this problem in order to reduce, if not eliminate, the biofilm (http://www.siditalia.it/ricerca/journal-club/piede-diabetico/404-new-molecular-techniques-to-study-the-skin-microbiota-of-diabetic-foot-ulcers).

Also well known in the dental field, bacterial biofilm is one of the main causes of two of the most common dental diseases, namely caries and periodontal disease.

Hybenx and Debacterol of Epien Medical Inc. are two products for professional dental use that rely on a chemical/physical action mechanism to remove the biofilm by dehydration: the matrix of the bacterial biofilm is coagulated by drying, contracting and detaching from the surface where it is strongly adhered. This chemical action facilitates the mechanical removal of the biofilm and allows the elimination of the bacteria. Hybenx is intended for the removal of bacterial plaque; Debacterol is dedicated to the treatment of ulcerative lesions.

Hybenx and Debacterol comprise a concentrated mixture of acidified sulphonated phenols; in particular, the dehydrating agent consists of 30-60% sulphonated phenols, 25-35% sulphuric acid, water.

### Problems of the background art

The above-mentioned products have a potent contact dehydrating action, as they contain high concentrations of sulphuric acid and sulphonic acids; sulphuric acid is essential in the formulation of sulphonated phenol-based products to achieve the desired dehydrating effect of the bacterial biofilm.

In fact, it is believed that, in these products, the sulphuric acid does not act as a dehydrator by means of a chemical dehydration reaction, but draws water from the contact surface by means of a solvation reaction (i.e., "self-dissolution"); in fact, the solvation reaction of sulphuric acid is favoured from a thermodynamic point of view.

As well as being useful for the dehydrating effect, the presence of sulphuric acid is actually a consequence of the industrial preparation technique of the product: sulphonated phenols are synthesized in the presence of concentrated sulphuric acid; at the conclusion of the reaction, the excess sulphuric acid used in the production plant is in the mixture with the synthetic products. Since sulphonated phenols are very low-melting liquids or solids, their separation from the sulphuric acid residue would be very difficult and very expensive.

The dehydrating power of acidified sulphonated phenol products is established in the formulation step, creating a solvation degree of the sulphate groups sufficient to achieve the desired effect, but such as to prevent a destructive dehydration of the tissue on which the preparation is applied.

The above-mentioned prior art products necessarily require the presence of sulphuric acid in order to achieve the desired effect.

It is believed that the presence of sulphonic derivatives in the products of the prior art is mainly based on the following reasons:
- they add viscosity to the preparation, which otherwise would be very fluid and difficult to use;
- they reduce the amount of sulphuric acid needed to achieve the dehydrating effect.

Lastly, the use of these products has several disadvantages of use: firstly, the product cannot be left in place for longer than 30-60 seconds, to avoid excessive dehydration of the contact tissue; the presence of sulphuric acid is responsible for irritation and burning in the application area and is therefore not appreciated by the patient.

### SUMMARY OF THE INVENTION

The Applicant has now formulated a pharmaceutical composition comprising non-phenolic (or alcohol-free) benzene sulphonic acids for use as a medicament, preferably suitable for external skin or mucosal disinfection.

The object of the present invention is therefore a pharmaceutical composition for use as a medicament comprising one or more compounds of formula (I) 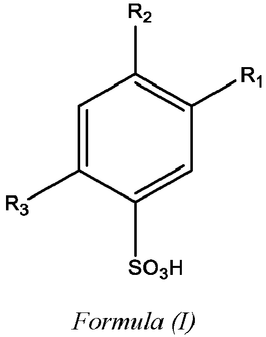
wherein at least one of the substituents R1, R2 and R3 is selected in the group consisting of: straight or branched C1-C4 alkyl groups, C1-C3 chain aliphatic ethers, aromatic ethers and halogens, and
the remaining substituent(s) among R1, R2 and R3 is hydrogen.

A further object of the present invention is a kit comprising the pharmaceutical composition, for use in the treatment of skin ulcers, in combination with devices suitable for the application and/or dosage of the pharmaceutical composition.

### Advantages of the invention

The compound of formula (I) selected by the Applicant is in solid or oily (highly viscous) form and can therefore be easily purified by removing excess sulphuric acid also industrially (for example by crystallization from water).

The product does not need to be formulated with sulphuric acid to achieve the desired effect; as better described in the detailed description of the invention, there are several alternatives in which the compound can be formulated.

Advantageously, the possibility of using carriers other than sulphuric acid allows to formulate less irritating products at the time of use and facilitate the industrial preparation steps of the pharmaceutical composition.

The absence of phenolic derivatives makes the product of the invention less toxic.

The smell of the product is more pleasant, by virtue of the absence of phenolic derivatives.

Lastly, the preparation may be coloured, by means of food dyes or pH indicators.

The product has a dehydrating action which occurs within minutes (3-5 minutes), thus making it easier to use the composition, without the urgency of having to remove the product within a few seconds.

### DETAILED DESCRIPTION OF THE INVENTION

For the purposes of the present invention, the expression "comprising" does not exclude the possibility that additional components other than those expressly listed may be present.

The compound of formula (I) belongs to the class of substituted benzene sulphonic acids, where the substituents R1, R2, R3 are respectively located in position - meta, - para and -ortho with respect to the sulphonic group.

The inventive composition comprises one or more compounds of formula (I), in which for each compound contained therein, at least one of substituents R1, R2 and R3 is selected from straight or branched C1-C4 alkyl groups, C1-C3 chain aliphatic ethers, aromatic ethers and halogens.

For the purposes of the present invention, straight alkyl groups are preferably selected from the group consisting of methyl, ethyl, propyl, butyl. Methyl is among the preferred straight C1-C4 alkyl groups for the purposes of the present invention.

Preferably, the branched C1-C4 alkyl groups are selected from the group consisting of isopropyl, tert-butyl, sec-butyl.

For the purposes of the present invention, C1-C3 aliphatic ether (-OR) may be straight or branched, preferably straight. Methoxyl ether is among the preferred C1-C3 aliphatic ethers for the purposes of the invention.

For the purposes of the present invention, aromatic ethers (-OAr) means both un-substituted aromatic ethers and substituted aromatic ethers.

It should be noted that phenyl ether is the preferred aromatic ether among the un-substituted aromatic ethers.

Preferably, the substituted aromatic ethers are selected from the group consisting of para-tolyether, meta-tolyether, ortho-tolyether, para-sulfonylphenyl ether.

According to a preferred embodiment, the suitable halogens for the purposes of the present invention are Bromine, Chlorine, Fluorine and Iodine, preferably Bromine and Chlorine. Among the halogens, Chlorine is the preferred substituent for the purposes of the invention.

According to a first embodiment, the pharmaceutical composition comprises an essentially pure compound of formula (I), where pure means a compound with a titre greater than 97% (w/w), preferably between 97% and 100% (w/w), preferably equal to 97%, 98%, 99% or 100% (w/w).

Preferably, the essentially pure compound of formula (I) is characterized as follows
- R2 is selected from the group consisting of straight or branched C1-C4 alkyl groups, C1-C3 aliphatic ethers and halogens, and
- R1 and R3 are independently selected among hydrogen and straight C1-C4 alkyl groups.

Preferably, in the essentially pure compound of formula (I), R2 is selected from straight C1-C4 alkyl groups and halogens; R1 and R3 are both hydrogen.

Preferably, the pure compound of formula (I) is selected between p-toluene sulphonic acid [Formula (Ia)] and p-chloro benzene sulphonic acid [Formula (Ib)]

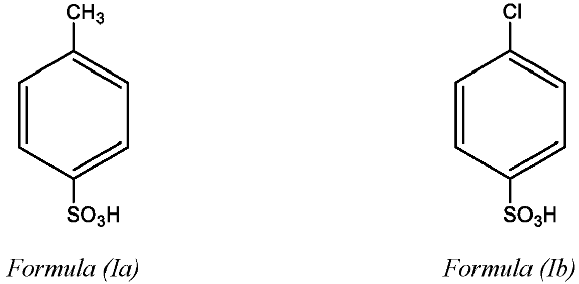

According to a second embodiment, the pharmaceutical composition of the invention comprises a mixture of compounds of formula (I).

Preferably, the pharmaceutical composition contains a mixture (N) comprising
- a first compound of formula (I) in which R2 is an un-substituted aromatic ether, R1 and R3 are hydrogen;
- a second compound of formula (I) in which R2 is a substituted aromatic ether, R1 and R3 are hydrogen.

Preferably, the first compound in the mixture (N) is 4-phenoxy benzenesulfonic acid [Formula (Im1)].

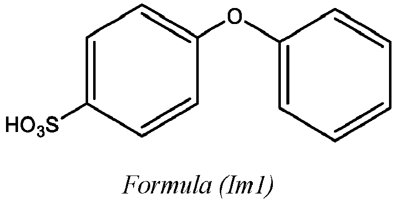

Preferably, the second compound of the mixture (N) is 4,4'-oxydibenzenesulfonic acid [Formula (Im2)].

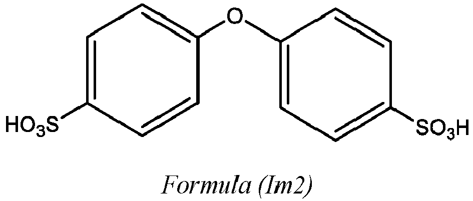

It should be noted that the mixture (N) is a mixture of mono- and di-sulphonate compounds in a ratio of 2:1; this ratio can be determined by 1H-NMR analysis.

In another preferred embodiment, the pharmaceutical composition comprises a mixture (M) containing
- a first compound of formula (I) in which R2 and R3 are independently selected from the group consisting of methyl, ethyl, propyl, butyl, isopropyl, tert-butyl and sec-butyl and R1 is hydrogen;
- a second compound of formula (I) in which R1 and R3 are independently selected from the group consisting of methyl, ethyl, propyl, butyl, isopropyl, tert-butyl and sec-butyl and R1 is hydrogen.

Preferably, the first compound of the mixture (M) is 2,4-dimethylbenzenesulfonic acid [Formula (In1)]; preferably the second compound of the mixture (M) is 2,5-dimethylbenzenesulfonic acid [Formula (In2)].

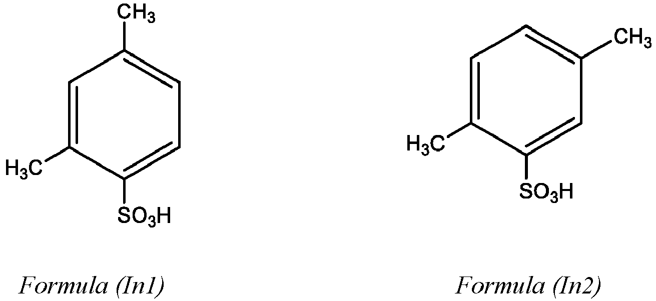

It should be noted that, according to the preferred embodiment, the pharmaceutical composition is intended for medical use, by topical application.

It should be noted that the inventive composition comprises the one or more compounds of formula (I) as the active ingredient; according to a preferred embodiment, the inventive composition comprises the one or more compounds of formula (I) as the only active ingredient.

It should be noted that the compounds of formula (I) are obtainable by synthetic techniques known to those skilled in the art and available in the literature (Corby et al., J. Chem. Research (S), 2002, 326-327); refer to the example embodiments for details.

Preferably, the pharmaceutical composition is for the treatment and external disinfection of skin ulcers.

Skin ulcers are losses of skin tissue, when the re-epithelialization process is unable, due to several factors, to regenerate the skin. Ulcers can occur in all skin areas in the human body.

The common symptom of all ulcerations is pain, linked to the exposure of the deep layers of the epithelium to inflammatory and irritative processes caused by the increased sensitivity to mechanical, chemical, infectious trauma.

Skin ulcers occur with symptoms which include: pain; swelling (oedema); redness; bleeding (https://www.humanitas.it/malattie/ulcera-cutanea).

Preferably, the skin ulcers for which the pharmaceutical composition is intended are selected from the group consisting of decubitus or pressure ulcers; diabetic foot ulcers, oral ulcers, vascular ulcers.

According to a preferred embodiment, the pharmaceutical composition of the invention comprises the compound of formula (I) in a concentration between 20% and 80% (w/w) by weight, based on the total weight of the pharmaceutical composition.

Preferably, the pharmaceutical composition comprises the compound of formula (I) in a concentration of 30% to 70% (w/w), preferably 35% to 65% (w/w), 40% to 60% (w/w), preferably 45% to 60% (w/w), preferably 45%, 46%, 47%, 48%, 49%, 50%, 51%, 52%, 53%, 54%, 55%, 56%, 57%, 58%, 59%, 60% (w/w).

The pharmaceutical composition comprises a vehicle selected from the group consisting of polyols, dimethyl sulfoxide (DMSO), aqueous solutions of micronized silica, and combinations of the foregoing.

The carrier allows the formulation of the active compound in a manner suitable for application; as already explained above, the compound of formula (I) has greater formulation versatility and can be conveyed in more delicate carriers, which do not have irritating properties like sulphuric acid.

It should be noted that preferably the carrier and the compound of formula (I) are combined in a weight ratio of 4:1 to 1:2, preferably 3:1 to 1:1.5, preferably 2.5:1 to 1:1.5.

### Dimethyl sulfoxide (DMSO)

According to a preferred embodiment, the carrier comprises pure DMSO or aqueous DMSO solutions at a concentration between 70% and 90%, preferably between 75% and 85%, preferably equal to 80%.

### Polyols

According to a preferred embodiment, the carrier comprises polyols selected from the group consisting of glycerol, ethylene glycols, propylene glycols, polyethylene glycols, and mixtures of the foregoing.

It should be noted that it is preferable that glycerin is used in combination with at least one other polyol as a carrier for the compositions of the invention.

Preferably, the carrier comprises a pure polyol or aqueous solution of one or more polyols.

In the case of aqueous solutions of one or more polyols, the concentration of polyol(s) is between 20% and 90% by weight of the total weight of the carrier.

Preferably, the carrier is composed of glycerin and 1,3-propanediol in 2.4:1 (2:1 v/v) weight ratio.

### Micronized silica aqueous solutions

According to a preferred embodiment, the carrier comprises aqueous solutions of micronized silica, preferably at a concentration between 0.1% and 5% by weight of the total carrier weight.

Preferably, the micronized silica is characterized by a specific surface area of 200 m²/g (Aerosil 200).

### Additional ingredients

According to a preferred embodiment, the pharmaceutical composition further comprises dyes useful for signalling possible contamination of the preparation and thereby signalling to the user that the pharmaceutical composition is no longer suitable for application on skin ulcerations.

Preferably, suitable dyes for the pharmaceutical composition of the invention are food grade dyes, pH indicators (such as methylene blue).

A further object of the invention is a kit for use in the external disinfection of skin ulcers, comprising the pharmaceutical composition and devices for the application and/or dosage of the pharmaceutical composition.

The devices for the application and/or dosage of the pharmaceutical composition of interest are those available in the state of the art and known to those skilled in the art, compatible with the carrier of use. Indicative and non-limiting examples of devices for application and/or dosage are pre-filled syringes; bottles or dropper caps; ampoules; spatulas; oral swabs.

### EXAMPLES

Illustrative and non-limiting examples of the pharmaceutical composition according to the invention are given below.

### Synthesis process

**Table 1**

| **Compound (CPD)** | **Structure** | **Notes** |
|---|---|---|
| **1** | 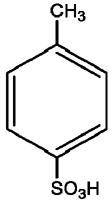 | *Solid* |
| **2** | 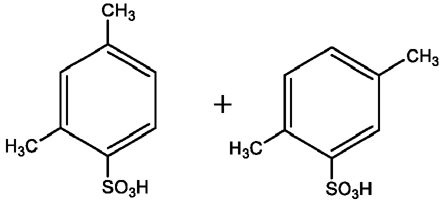 | Unsolved mixture (M) of two isomers *Viscous oil* |
| **3** | 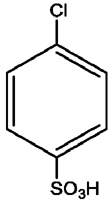 | *Solid* |
| **4** | 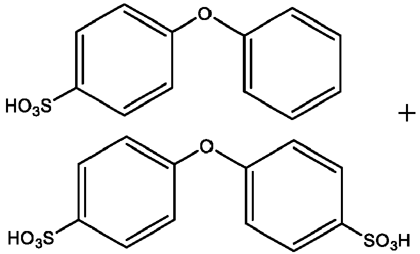 | Unsolved mixture (N) *Viscous oil* |

Trifluoroacetic anhydride (1.48 mL, 10.50 mmol) was slowly added to sulphuric acid at a temperature of about 0° (278 µL, 5.00 mmol). The thus-obtained emulsion was mixed at 0°C until a clear solution (2 hours) was obtained. The un-sulphonated raw material (toluene, xylene, p-chlorobenzene or diphenylether for la, M, Ib and N respectively; 5.00 mmol) was added to the previously obtained solution, stirring at room temperature for 30 minutes.

After the addition of water (140 µL, 7.50 mmol), the mixture was kept under stirring for a further 30 minutes.

The reaction mixture was concentrated under vacuum so as to (independently) obtain compounds 1 to 3.

In the case of the mixture (N), it was necessary to perform an intermediate purification step: after removing the volatile components, the mixture was taken up with n-hexane (10 mL) and mixed for 30 minutes. The hexane supernatant was removed, creating a red viscous oil and leading to the isolation of the mixture (N) (as a mixture of mono-and di-sulphonate compounds in a 2:1 ratio as determined by the 1H-NMR analysis).

The compounds were characterized by proton nuclear mass spectrometry and magnetic resonance imaging (H-NMR).

All of the synthesized compounds were formulated in the following carriers:

**Table 2**

| **Carrier** | **Composition (%v/v)** |
|---|---|
| **B** | 100% DMSO |
| **C** | 100% glycerol |
| **D** | 80% glycerol, 20% H₂O |
| **E** | Micronized silica 2% in H₂O |
| **F** | 1,3 -propanediol :glycerine (w/w = 1:2.4) |

On the basis of such carriers, the following compositions were prepared:

**Table 3**

| **Mixture** | **CPD no. (mg)** | **Carrier (µL)** | **Mixture** | **CPD no. (mg)** | **Carrier (µL)** |
|---|---|---|---|---|---|
| B1 | 1 (150) | B (150) | D3 | 3 (150) | D (150) |
| B2 | 2 (150) | | D4 | 4 (150) | |
| B3 | 3 (150) | | E1 | 1 (150) | E (150) |
| B4 | 4 (150) | | E2 | 2 (150) | |
| C1 | 1 (150) | C (150) | E3 | 3 (150) | |
| C2 | 2 (150) | | E4 | 4 (150) | |
| C3 | 3 (150) | | F3 | 1 (86.4) | F (150) |
| C4 | 4 (150) | | F1 | 1 (105.5) | |
| D1 | 1 (150) | D (150) | F2 | 1(95.8) | |
| D2 | 2 (150) | | | | |

The efficacy of the compositions of interest was preliminarily tested in vitro on bacterial species relevant in the contamination of skin ulcers: *Staphylococcus aureus, Pseudomonas aeruginosa, Proteus mirabilis.* The bacterial cultures were maintained under standard conditions and then seeded (1x10⁴ CFU/well) in 96-well plates and incubated at 37°C for 48 hours to ensure biofilm formation.

The biofilms thus obtained were washed and incubated with 80 micro-litres of the mixtures at room temperature for 5, 15, 30, 60, 150, 300, 600 or 900 seconds.

In order to test the immediate effect of the mixtures, at the end of incubation the biofilms were harvested, washed and sown on suitable solid medium. The cultures were incubated at 37°C and monitored for the appearance of bacterial colonies for up to 72 hours.

In order to test the effectiveness of the mixtures over time, the biofilms were treated as above and then washed and incubated with fresh culture broth. The biofilms were incubated for an additional 72 hours. At the end of this incubation, the biofilms were harvested, washed and sown on suitable solid medium. The cultures were incubated at 37°C and monitored for the appearance of bacterial colonies for up to 72 hours.

## Claims

1. Pharmaceutical composition for use as a medicament comprising one or more formula compounds (I) 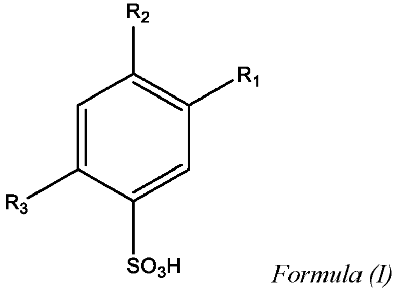
wherein at least one of the substituents R1, R2 and R3 is selected from the group consisting of: straight or branched C1-C4 alkyl groups, C1-C3 chain aliphatic ethers, aromatic ethers and halogens, and
the remaining substituent(s) among R1, R2 and R3 is hydrogen.

2. Pharmaceutical composition for use according to claim 1, wherein
R2 is selected from the group consisting of straight or branched C1-C4 alkyl groups, C1-C3 aliphatic ethers and halogens, and
R1 and R3 are independently selected among hydrogen, straight C1-C4 alkyl groups.

3. Pharmaceutical composition for use according to claim 1, comprising
- a first compound of formula (I) in which R2 is an un-substituted aromatic ether, R1 and R3 are hydrogen;
- a second compound of formula (I) in which R2 is a substituted aromatic ether, R1 and R3 are hydrogen.

4. Pharmaceutical composition for use according to claim 1, comprising
- a first compound of formula (I) in which R2 and R3 are independently selected in the group consisting of methyl, ethyl, propyl, butyl, isopropyl, ter-butyl, sec-butyl, and R1 is hydrogen;
- a second compound of formula (I) in which R1 and R3 are independently selected in the group consisting of methyl, ethyl, propyl, butyl, isopropyl, ter-butyl, sec-butyl, and R2 is hydrogen.

5. Pharmaceutical composition for use according to any one of claims from 1 to 4, in the external disinfection of skin ulcers.

6. Pharmaceutical composition for use according to claim 5, wherein skin ulcers are selected in the group consisting of decubitus or pressure ulcers; diabetic foot ulcers, oral ulcers, vascular ulcers.

7. Pharmaceutical composition for use according to any one of claims from 1 to 6, wherein the content of the compound of formula (I) ranges between 20% and 80% by weight of the total weight of the pharmaceutical composition.

8. Pharmaceutical composition for use according to any one of claims from 1 to 7, comprising a carrier selected from the group consisting of polyols, dimethyl sulfoxide (DMSO), micronized silica solutions and combinations of the foregoing.

9. Pharmaceutical composition for use according to claim 8, wherein the carrier and the compound of formula (I) are in a weight ratio ranging between 4:1 and 1:2.

10. Kit for use in the external disinfection of skin ulcers, comprising a pharmaceutical composition according to any one of claims from 1 to 9 and devices for application and/or dosage of the pharmaceutical composition.
